# EUROPEAN PATENT SPECIFICATION

(11) **EP 1 866 426 B1**
(45) Date of publication and mention of the grant of the patent: **24.12.2008**
(21) Application number: 06720921.3
(22) Date of filing: 22.02.2006
(51) Int. Cl.: C12P 19/04

(54) **PREPARATION OF SLOWLY DIGESTIBLE CARBOHYDRATES BY SSMB CHROMATOGRAPHY**
HERSTELLUNG LANGSAM VERDAUBARER KOHLENHYDRATE DURCH SSMB-CHROMATOGRAPHIE
PREPARATION DE GLUCIDES A DIGESTION LENTE PAR CHROMATOGRAPHIE SSMB

(30) Priority: 17.03.2005 US 83347
(43) Date of publication of application: 19.12.2007
(73) Proprietor: Tate & Lyle Ingredients Americas, Inc., Decatur, IL 62521 (US)
(72) Inventor: HOFFMAN, Andrew, J., Decatur, IL 62521 (US); ARMENTROUT, Richard, W., Decatur, IL 62521 (US); LIU, Chi-Li, Decatur, IL 62526 (US)
(74) Representative: Smyth, Gyles Darren
(86) International application number: PCT/US2006/006037
(87) International publication number: WO 2006/101648

(56) References cited:
- US-B1- 6 406 547
- US-B2- 6 663 780
- GOULAS ET AL: "Purification of oligosaccharides by nanofiltration" JOURNAL OF MEMBRANE SCIENCE, vol. 209, 2002, pages 321-335, XP002384101
- GOULAS ET AL: "Fractionation of oligosaccharides by nanofiltration" JOURNAL OF THE SCIENCE OF FOOD AND AGRICULTURE, vol. 83, 2003, pages 675-680, XP004382364
- BAUDOUIN ET AL: "Desugarization of low purity syrups and molasses: The sequential simulated moving bed (SSMB) is a new step of progress in the development of chromatographic separation for two fraction systems" CITS - INTERNATIONAL COMMISSION FOR SUGAR TECHNOLOGY - 22ND GENERAL ASSEMBLY, MAY 2003, MADRID, SPAIN, 2003, page 1, XP002384235 Retrieved from the Internet: URL:http://www.cits-sugar.org/cits/cits_ab stracts/26.htm> [retrieved on 2006-06-07]
- KANEKO ET AL: "Characterization of acid-stable glucose isomerase from Streptomyces sp., and development of single-step processes for high-fructose corn sweetener (HFCS) production" BIOSCIENCE, BIOTECHNOLOGY AND BIOCHEMISTRY, vol. 64, 2000, pages 940-947, XP002384236

## Description

### BACKGROUND OF THE INVENTION

A variety of carbohydrates are used in food products. Corn starch is one example. The carbohydrates in food products typically are digested in the human stomach and small intestine. Dietary fiber in food products, in contrast, is generally not digested in the stomach or small intestine, but may be at least partially bioconverted by microorganisms in the large intestine.

There is an interest in developing ingredients that are suitable for use in food products and that are either non-digestible or only digestible to a limited extent, in order to enhance the dietary fiber content or reduce the caloric content of the food. These modifications are thought to have certain health benefits.

There is a need for edible materials which have a reduced content of easily digestible carbohydrates, and which can be used in place of or in addition to conventional carbohydrate products in foods.

The present invention therefore refers to a process for making an oligosaccharide composition, comprising the use of sequential simulated moving bed chromatography.

Sequential simulated moving bed chromatography has, for example, been used for fractionation of molasses; see US 6,663,780 B2.

### SUMMARY OF THE INVENTION

One aspect of the invention is a process for making an oligosaccharide composition. The process comprises producing an aqueous composition that comprises at least one oligosaccharide and at least one monosaccharide by saccharification of starch; fractionating the aqueous composition by a method comprising sequential simulated moving bed chromatography to form a monosaccharide-rich stream and an oligosaccharide-rich stream; and recovering the oligosaccharide-rich stream. The oligosaccharide-rich stream is slowly digestible by the human digestive system. "Slowly digestible" as the term is used herein means that a substantial quantity (e.g., at least about 50% on a dry solids basis, and in some cases at least about 75%, or at least about 90%) of the carbohydrates present in the stream are either not digested at all in the human stomach and small intestine, or are only digested to a limited extent.

Both in vitro and in vivo tests can be performed to estimate rate and extent of carbohydrate digestion in humans. The "Englyst Assay" is an in vitro enzyme test that can be used to estimate the amounts of a carbohydrate ingredient that are rapidly digestible, slowly digestible or resistant to digestion (European Journal of Clinical Nutrition (1992) Volume 46 (Suppl.2), pages S33-S50). Thus, any reference herein to "at least about 50% by weight on a dry solids basis" of a material being slowly digestible means that the sum of the percentages that are classified as slowly digestible or as resistant by the Englyst assay totals at least about 50%.

In one embodiment of the process, the aqueous composition that is produced by saccharification of starch, followed by isomerization, comprises dextrose, fructose, and a mixture of oligosaccharides. This aqueous composition is fractionated to separate it into the monosaccharide-rich permeate stream and the oligosaccharide-rich retentate stream. The oligosaccharide-rich stream can comprise at least about 50% by weight oligosaccharides on a dry solids basis, or in some cases at least about 90%. In certain embodiments of the process, the oligosaccharide-rich stream will still comprise a minor amount of dextrose and fructose. "A minor amount" is used herein to mean less than 50% by weight on a dry solids basis.

The process, can, in some embodiments, also include one or more of the following steps: (1) contacting the oligosaccharide-rich stream with an isomerization enzyme, such that at least some of the dextrose is converted to fructose, thereby producing an isomerized oligosaccharide-rich stream; (2) membrane filtering the oligosaccharide-rich stream to produce a second monosaccharide-rich stream and a second oligosaccharide-rich stream that comprises more than about 90% by weight oligosaccharides on a dry solids basis as well as a minor amount of monosaccharides; (3) hydrogenating the oligosaccharide-rich stream to convert at least some of the monosaccharides therein to alcohols, thereby producing a hydrogenated oligosaccharide-rich stream; (4) contacting the oligosaccharide-rich stream with a glucosidase enzyme to create a reversion product such that at least some of any residual monosaccharides present in the stream are covalently bonded to oligosaccharides or other monosaccharides; and (5) reducing the color of the oligosaccharide-rich stream by contacting it with activated carbon.

Also disclosed is an edible carbohydrate composition that comprises a major amount of oligosaccharides on a dry solids basis, and that is slowly digestible by the human digestive system. This composition can be produced by the above-described process. "Major amount" is used herein to mean at least 50% by weight on a dry solids basis.

In one embodiment, the edible carbohydrate composition is produced by a process as described above. In one particular embodiment, the oligosaccharide rich stream has a solids content not less than 70.0 percent mass/mass (m/m), and a reducing sugar content (dextrose equivalent), expressed as D-glucose, that is not less than 20.0 percent m/m caicuiated on a dry basis. This composition can be classified as corn syrup under food labeling regulations. In another alternative, the oligosaccharide rich stream has a solids content not less than 70.0 percent mass/mass (m/m), and reducing sugar content (dextrose equivalent), expressed as D-glucose, less than 20.0 percent m/m calculated on a dry basis. This can be classified as maltodextrin under food labeling regulations.

Further disclosed is a method of preparing a food product. The method comprises providing a food composition suitable for combination with a carbohydrate material, and combining the food composition with an edible carbohydrate composition that is slowly digestible, as described above.

### BRIEF DESCRIPTION OF THE DRAWING

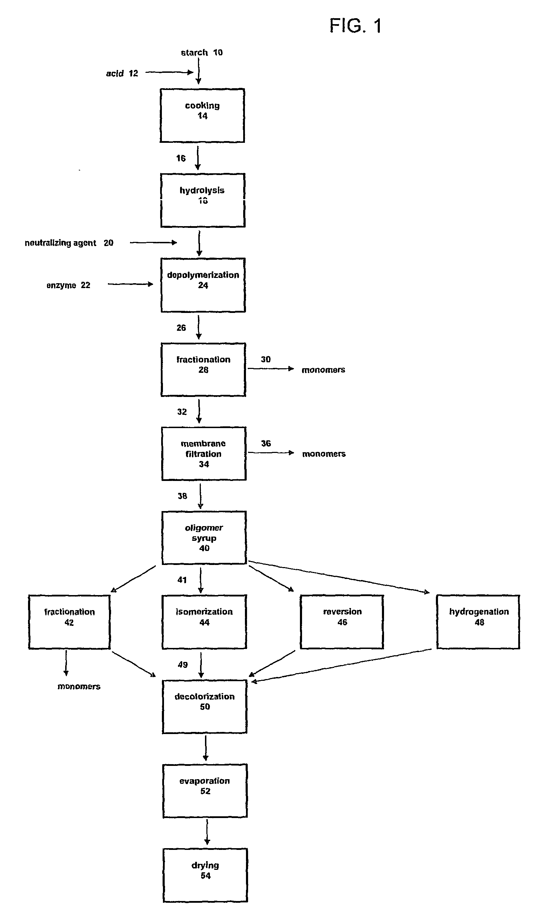

Figure 1 is process flow diagram comprising chromatographic fractionation.

### DESCRIPTION OF SPECIFIC EMBODIMENTS

One aspect of the present invention is a process for making a slowly digestible carbohydrate composition that is suitable for use in foods. It should be understood that the term "food" is used in a broad sense herein to include a variety of other substances that can be ingested by humans, such as beverages and medicinal capsules or tablets.

The process can begin with a starch, for example a vegetable starch. Conventional corn starch is one suitable example. The process will generally operate more efficiently if the beginning starch has a relatively high purity. In one embodiment, the high purity starch contains less than 0.5% protein on a dry solids basis. Although some of the following discussion focuses on corn, it should be understood that the present invention is also applicable to starches derived from other sources, such as potato and wheat, among others.

As shown in Figure 1, the starch 10 can have acid 12 added to it and can then be gelatinized 14 in a starch cooker, for example in a jet cooker in which starch granules are contacted with steam. In one version of the process, the starch slurry, adjusted to a pH target of 3.5 by addition of sulfuric acid, is rapidly mixed with steam in a jet cooker and held at 149 to 152 °C (300 to 305 °F) for 4 minutes in a tail line. The gelatinized starch 16 is hydrolyzed 18 by exposure to acid at high temperature during jet cooking. The hydrolysis reduces the molecular weight of the starch and generates an increased percentage of monosaccharides and oligosaccharides in the composition. (The term "oligosaccharides" is used herein to refer to saccharides comprising at least two saccharide units, for example saccharides having a degree of polymerization (DP) of about 2-30.) A neutralizing agent 20, such as sodium carbonate, can be added to stop the acid hydrolysis, and then the composition can be further depolymerized 24 by contacting it with a hydrolytic enzyme 22. Suitable enzymes include alpha amylases such as Termamyl, which is available from Novozymes. This enzymatic hydrolysis further increases the percentage of monosaccharides and oligosaccharides present in the composition. The overall result of the hydrolysis by acid and enzyme treatment is to saccharify the starch. The saccharified composition can be isomerized to change the monosaccharide profile, for example to increase the concentration of fructose.

The saccharified composition 26 is then purified by chromatographic fractionation 28. In the method of the invention, which employs a sequential simulated moving bed (SSMB) chromatography procedure, a solution of mixed saccharides is pumped through a column filled with resin beads. Depending on the chemical nature of the resin, some of the saccharides interact with the resin more strongly leading to a retarded flow through the resin compared to saccharides that interact with the resin more weakly. This fractionation can produce one stream 30 that has a high content of monosaccharides, such as dextrose and fructose. High fructose corn syrup is an example of such a stream. The fractionation also produces a raffinate stream 32 that has a relatively high concentration of oligosaccharides (e.g., about 5 - 15 % oligosaccharides on a dry solids basis (d.s.b.)) and also contains a smaller concentration of monosaccharides such as dextrose and fructose. Although the term "stream" is used herein to describe certain parts of the process, it should be understood that the process of the present invention is not limited to continuous operation. The process can also be performed in batch or semi-batch mode.

The raffinate 32 can be further fractionated by membrane filtration 34, for example by nanofiltration, optionally with diafiltration. For example, these filtration steps can be performed using a Desal DK spiral wound nanofiltration cartridge at about 500 psi of pressure and at 40 - 60 degrees centigrade temperature. The fractionation described in step 34 could also be accomplished by sequential simulated moving bed chromatography (SSMB). The membrane filtration produces a permeate 36 which comprises primarily monosaccharides, and a retentate 38 which comprises primarily oligosaccharides. ("Primarily" as used herein means that the composition contains more of the listed component than of any other component on a dry solids basis.) The permeate 36 can be combined with the monomer stream 30 (e.g., high fructose corn syrup). The permeate is a monosaccharide-rich stream and the retentate is an oligosaccharide-rich stream. In other words, the nanofiltration concentrates the oligosaccharides in the retentate and the monosaccharides in the permeate, relative to the nanofiltration feed.

The retentate 38, which can be described as an oligosaccharide syrup 40, can have a sufficiently high content of oligosaccharides that are slowly digestible (e.g., at least about 50% by weight d.s.b., or in some cases at least about 90%) so that it can be dried or simply evaporated to a concentrated syrup and used as an ingredient in foods. However, in many cases, it will be useful to further process and purify this composition. Such purification can include one or more of the following steps. (Although Fig. 1 shows four such purification steps 42, 44, 46, and 48 as alternatives, it should be understood that two or more of these steps could be used in the process.)

One option is to subject the oligomers syrup 40 to another fractionation 42, such as a membrane filtration, for example a second nanofiltration, in order to remove at least some of the residual monosaccharides, such as fructose and dextrose. Suitable nanofiltration conditions and equipment are as described above. This nanofiltration produces a permeate, which is a second monosaccharide-rich stream, which can be combined with the monomer stream 30. Alternatively, the further fractionation 42 could be done by chromatographic separation, for example, by simulated mixed-bed chromatography.

Another option is to isomerize 44 the syrup 41 by contacting it with an enzyme such as glucose isomerase. This will convert at least some of the residual dextrose present into fructose, which may be more valuable in certain situations.

Another option is to treat the syrup with an enzyme to cause reversion or repolymerization 46, in which at least some of the relatively small amounts of monosaccharides that are still present are covalently bonded to other monosaccharides or to oligosaccharides, thereby reducing the residual monomer content of the syrup even further. Suitable enzymes for use in this step include glucosidases, such as amylase, glucoamylase, transglucosidase, and pullulanase. Cellulase enzymes may produce valuable reversion products for some applications.

Yet another option is to hydrogenate 48 the syrup to convert at least some of any residual monosaccharides to the corresponding alcohols (e.g., to convert dextrose to sorbitol). When hydrogenation is included in the process, it will typically (but not necessarily) be the final purification step.

The purified oligomer syrup 49 produced by one or more of the above purification steps can then be decolorized 50. Decolorization can be done by treatment with activated carbon followed by microfiltration, for example. In continuous flow systems, syrup streams can be pumped through columns filled with granular activated carbon to achieve decolorization. The decolorized oligomer syrup can then be evaporated 52, for example to about greater than about 70% dry solids (d.s.), giving a product that comprises a high content of oligosaccharides (e.g., greater than 90% by wt d.s.b., and in some instances greater than 95%), and a correspondingly low monosaccharide content. The product comprises a plurality of saccharides which are slowly or incompletely digested by humans, if not totally indigestible. These sugars can include isomaltose, panose and branched oligomers having a degree of polymerization of four or greater.

The process conditions can be modified to recover the majority of the maltose in the feed either in the monomer-rich streams (30, 36) or in the oligomer product stream. For example, a nanofiltration membrane with a slightly more open pore size, such as Desal DL, operating at less than 500 psi pressure can be used to increase the amount of maltose in monomer-rich streams.

The product is suitable as an ingredient for foods, and is slowly digestible by the human digestive system. As mentioned above, some components of the product can be substantially entirely indigestible in the human stomach and small intestine. Depending on the starch source used, the product can be classified in some embodiments as corn syrup or wheat syrup, as those terms are used in food labeling. In cases where more open pore sizes are used in nanofiltration, a higher molecular weight oligomer syrup product classified as a maltodextrin can be obtained.

The oligosaccharide-containing syrup produced by the process can be added to foods as replacement or supplement for conventional carbohydrates. Specific examples of foods in which the syrup can be used include processed foods such as bread, cakes, cookies, crackers, extruded snacks, soups, frozen desserts, fried foods, pasta products, potato products, rice products, corn products, wheat products, dairy products, yogurts, confectionaries, hard candies, nutritional bars, breakfast cereals, and beverages. A food product containing the oligosaccharide syrup will have a lower glycemic response, lower glycemic index, and lower glycemic load than a similar food product in which a conventional carbohydrate, such as corn starch, is used. Further, because at least some of the oligosaccharides are either only digested to a very limited extent or are not digested at all in the human stomach or small intestine, the caloric content of the food product is reduced. The syrup is also a source of soluble dietary fiber.

The process described herein takes advantage of a fraction of the saccharide syrup (e.g., stream 26 in Fig. 1) that is resistant to saccharification. By separating this material as a purified product, it can be employed for its own useful properties, rather than being an undesirable by-product in syrups that are primarily monosaccharides, such as high fructose corn syrup. Removal of a greater percentage of the oligosaccharides from the high fructose corn syrup allows that product to be made purer (i.e., with a higher concentration of dextrose and fructose) and thus more valuable.

### Example 1 (comparative)

Raffinate syrup was obtained from a plant in which corn starch was being processed into high fructose corn syrup. The raffinate was produced by a chromatographic separation, and comprised primarily fructose and dextrose. The raffinate was subjected to nanofiltration using a Desal DK1812C-31D nanofiltration cartridge at about 500 psi (34,5 x 10⁵ N/m²) of pressure and at a temperature of 40-60°C. The retentate from the nanofiltration was decolorized with activated charcoal, and then evaporated to approximately 80% dry solids. A saccharide analysis of the dry product was performed by HPAE-PAD chromatography, and the results are shown in Table 1.

**Table 1**

| Component | Wt % d.s.b. |
|---|---|
| dextrose | 38.9% |
| fructose | 6.1 % |
| isomaltose | 14.3% |
| maltose | 10.5% |
| maltotriose | 0.3% |
| panose | 9.5% |
| linear higher saccharides | 0.0% |
| nonlinear higher saccharides | 20.4% |

This material, termed Light Raffinate, was tested for digestibility using an Englyst assay. About 600 mg of carbohydrate d.s.b was added to 20 mL of 0.1 M sodium acetate buffer in a test tube. The contents were mixed and then heated to about 92 °C for 30 minutes, then cooled to 37 °C. Then 5 mL of enzyme solution was added to the test tube and it was agitated by shaking in a water bath at 37 °C. Small samples were removed at both 20 min and 120 min. The enzyme was inactivated, the samples were filtered and measured for digestibility using a glucose test from YSI Inc. A Heavy Raffinate, processed in a separate but similar nanofiltration operation, was also tested using the same assay. The Heavy Raffinate contained 25-35% dry solids, as opposed to 15-25% dry solids for the Light Raffinate, but both had approximately the same percentage of low molecular weight saccharides. A cooked potato starch, which had not been nanofiltered, was also tested as a comparison. The results of the digestibility assay and a saccharide analysis are shown in Table 2. Cooked potato starch is included in Table 2 for comparison. All percentages in Table 2 are on a d.s.b.

**Table 2**

| material | % rapidly | % slowly | % resistant | % mono- | % oligo- |
|---|---|---|---|---|---|
| | digestible | digestible | | saccharides | saccharides |
| | | | | (by HPAE) | (by HPAE) |
| Light raffinate | 45 | 3 | 52 | 45 | 55 |
| Heavy raffinate | 41 | 3 | 56 | 44 | 56 |
| Potato starch | 78 | 11 | 11 | 44 | 56 |
| (cooked) | | | | | |

There was an excellent correlation between the percentage of oligosaccharides in the material and the percentage of the material that was resistant to digestion.

### Example 2 (comparative)

About 1,025 L of raffinate syrup at 21.4% dry solids was obtained from a plant in which corn starch was being processed into high fructose corn syrup. The raffinate was produced by a chromatographic separation, and comprised primarily fructose and dextrose. The raffinate was subjected to nanofiltration using two Desal NF3840C-50D nanofiltration cartridges at about 500 psi of pressure and at a temperature of 40-60°C. After the starting volume was reduced by about a factor of 20, the retentate was subjected to about 2 volumes of constant volume diafiltration using DI water. After diafiltration, 27.6 kg of retentate product (at 33.8% ds) was collected. This material was decolorized with activated carbon (0.5% by weight of syrup solids) by stirring in a refrigerator overnight. This slurry was sterilized by filtration through a 0.45 micron hollow fiber filtration cartridge, and evaporated in parts to an average concentration of about 73% ds.

A saccharide analysis of the dry product was performed by HPAE-PAD chromatography, and the results are shown in Table 3.

**Table 3**

| Component | Wt % d.s.b. |
|---|---|
| dextrose | 4.5% |
| fructose | 0.9% |
| isomaltose | 20.6% |
| maltose | 23.5% |
| maltotriose | 0.4% |
| panose | 20.9% |
| linear higher saccharides | 0.0% |
| nonlinear higher saccharides | 29.1% |

## Claims

1. A process for making an oligosaccharide composition, comprising:
producing an aqueous composition that comprises at least one oligosaccharide and at least one monosaccharide by saccharification of starch;
fractionating the aqueous composition by a method comprising
sequential simulated moving bed chromatography to form a monosaccharide-rich stream and an oligosaccharide-rich stream; and
recovering the oligosaccharide-rich stream.

2. The process of claim 1, wherein the aqueous composition comprises dextrose, fructose, and a mixture of oligosaccharides.

3. The process of claim 1, wherein the oligosaccharide-rich stream comprises at least about 50% by weight oligosaccharides on a dry solids basis.

4. The process of claim 3, wherein the oligosaccharide-rich stream comprises at least about 90% by weight oligosaccharides on a dry solids basis.

5. The process of claim 1, wherein the fractionation comprises membrane filtering.

6. The process of claim 1, wherein the fractionation comprises nanofiltration.

7. The process of claim 1, wherein the oligosaccharide-rich stream comprises a minor amount of dextrose and fructose, and wherein the process further comprises contacting the oligosaccharide-rich stream with an isomerisation enzyme such that at least some of the dextrose is converted to fructose, thereby producing an isomerized oligosaccharide-rich stream.

8. The process of claim 1, further comprising membrane filtering the oligosaccharide-rich stream to produce a second monosaccharide-rich stream and a second oligosaccharide-rich stream.

9. The process of claim 8, wherein the second oligosaccharide-rich stream comprises more than about 90% by weight oligosaccharides on a dry solids basis.

10. The process of claim 1, wherein the oligosaccharide-rich stream comprises a minor amount of monosaccharides, and wherein the process further comprises hydrogenating the oligosaccharide-rich stream to convert at least some of the monosaccharides therein to alcohols, thereby producing a hydrogenated oligosaccharide-rich stream.

11. The process of claim 8, wherein the second oligosaccharide-rich stream comprises a minor amount of monosaccharides, and wherein the process further comprises hydrogenating the second oligosaccharide-rich stream to convert at least some of the monosaccharides therein to alcohols, thereby producing a hydrogenated oligosaccharide-rich stream

12. The process of claim 1, further comprising contacting the oligosaccharide-rich stream with a glucosidase enzyme such that at least some of any residual monosaccharides present in the stream are covalently bonded to oligosaccharides or other monosaccharides.

13. The process of claim 1, further comprising reducing the color of the oligosaccharide-rich stream by contacting it with activated carbon.

14. The process of claim 1, wherein the oligosaccharide-rich stream is slowly digestible by the human digestive system.

15. The process of claim 1, wherein the fractionation comprises nanofiltration, the aqueous composition comprises dextrose, fructose, and a mixture of oligosaccharides, and the oligosaccharide-rich stream comprises a minor amount of dextrose and fructose, and wherein the process further comprises at least one of the following:
contacting the oligosaccharide-rich stream with an isomerization enzyme such that at least some of the dextrose is converted to fructose;
membrane filtering the oligosaccharide-rich stream;
hydrogenating the oligosaccharide-rich stream to convert at least some of the monosaccharides therein to alcohols;
contacting the oligosaccharide-rich stream with a glucosidase enzyme to create a reversion product such that at least some of any residual monosaccharides present in the stream are covalently bonded to oligosaccharides or other monosaccharides; and
reducing the color of the oligosaccharide-rich stream by contacting it with activated carbon;
wherein the oligosaccharide-rich stream is slowly digestible by the human digestive system.

## Patentansprüche

1. Verfahren zur Herstellung einer Oligosaccharid-Zusammensetzung, umfassend:
Herstellung einer wässrigen Zusammensetzung, welche mindestens ein Oligosaccharid und mindestens ein Monosaccharid umfasst, durch Saccharifikation von Stärke,
Fraktionierung der wässrigen Zusammensetzung durch ein Verfahren, umfassend
simulierte Gegenstrom(SSMB)-Chromatographie, um einen Monosaccharid-reichen Strom und einen Oligosaccharid-reichen Strom zu bilden,
und
Gewinnung des Oligosaccharid-reichen Stroms.

2. Das Verfahren nach Anspruch 1, wobei die wässrige Zusammensetzung Dextrose, Fruktose und eine Mischung von Oligosacchariden umfasst.

3. Das Verfahren nach Anspruch 1, wobei der Oligosaccharid-reiche Strom mindestens etwa 50 Gew.-% Oligosaccharide auf Grundlage der Trockenfeststoffe umfasst.

4. Das Verfahren nach Anspruch 3, wobei der Oligosaccharid-reiche Strom mindestens etwa 90 Gew.-% Oligosaccharide auf Grundlage der Trockenfeststoffe umfasst.

5. Das Verfahren nach Anspruch 1, wobei die Fraktionierung Membranfiltration umfasst.

6. Das Verfahren nach Anspruch 1, wobei die Fraktionierung Nanofiltration umfasst.

7. Das Verfahren nach Anspruch 1, wobei der Oligosaccharid-reiche Strom eine geringe Menge Dextrose und Fruktose umfasst und wobei das Verfahren weiterhin das Inkontaktbringen des Oligosaccharid-reichen Stroms mit einem Isomerisationsenzym umfasst, so dass mindestens ein Teil der Dextrose in Fruktose umgewandelt wird, wodurch ein isomerisierter Oligosaccharid-reicher Strom hergestellt wird.

8. Das Verfahren nach Anspruch 1, welches weiterhin Membranfiltration des Oligosaccharid-reichen Stroms umfasst, um einen zweiten Monosaccharid-reichen Strom und einen zweiten Oligosaccharid-reichen Strom herzustellen.

9. Das Verfahren nach Anspruch 8, wobei der zweite Oligosaccharid-reiche Strom mehr als etwa 90 Gew.-% Oligosaccharide auf Grundlage der Trockenfeststoffe umfasst.

10. Das Verfahren nach Anspruch 1, wobei der Oligosaccharid-reiche Strom eine geringe Menge Monosaccharide umfasst und wobei das Verfahren weiterhin eine Hydrierung des Oligosaccharid-reichen Stroms umfasst, um mindestens einen Teil der Monosaccharide darin in Alkohole umzuwandeln, wodurch ein hydrierter Oligosaccharid-reicher Strom hergestellt wird.

11. Das Verfahren nach Anspruch 8, wobei der zweite Oligosaccharid-reiche Strom eine geringe Menge Monosaccharide umfasst und wobei das Verfahren weiterhin eine Hydrierung des zweiten Oligosaccharid-reichen Stroms umfasst, um mindestens einen Teil der Monosaccharide darin in Alkohole umzuwandeln, wodurch ein hydrierter Oligosaccharid-reicher Strom hergestellt wird.

12. Das Verfahren nach Anspruch 1, welches weiterhin das Inkontaktbringen des Oligosaccharid-reichen Stroms mit einem Glucosidase-Enzym umfasst, so dass mindestens ein Teil jeglicher verbleibenden Monosaccharide, die im Strom vorhanden sind, kovalent an Oligosaccharide oder andere Monosaccharide gebunden werden.

13. Das Verfahren nach Anspruch 1, welches weiterhin das Reduzieren der Farbe vom Oligosaccharid-reichen Strom umfasst durch Inkontaktbringen von diesem mit Aktivkohle.

14. Das Verfahren nach Anspruch 1, wobei der Oligosaccharid-reiche Strom langsam verdaubar ist durch das menschliche Verdauungssystem.

15. Das Verfahren nach Anspruch 1, wobei die Fraktionierung Nanofiltration umfasst, die wässrige Zusammensetzung Dextrose, Fruktose und eine Mischung von Oligosacchariden umfasst, und der Oligosaccharid-reiche Strom eine geringe Menge an Dextrose und Fruktose umfasst, und worin das Verfahren weiterhin mindestens einen der folgenden Schritte umfasst:
Inkontaktbringen des Oligosaccharid-reichen Stroms mit einem Isomerisationsenzym, so dass mindestens ein Teil der Dextrose in Fruktose umgewandelt wird;
Membranfiltration des Oligosaccharid-reichen Stroms;
Hydrierung des Oligosaccharid-reichen Stroms, um mindestens einen Teil der Monosaccharide darin in Alkohole umzuwandeln;
Inkontaktbringen des Oligosaccharid-reichen Stroms mit einem Glucosidase-Enzym, um ein Reversionsprodukt zu bilden, so dass mindestens ein Teil jeglicher verbleibenden Monosaccharide, welche im Strom vorhanden sind, kovalent an Oligosaccharide oder andere Monsaccharide gebunden werden, und
Reduzieren der Farbe vom Oligosaccharid-reichen Strom durch Inkontaktbringen von diesem mit Aktivkohle;
wobei der Oligosaccharid-reiche Strom langsam verdaubar ist durch das menschliche Verdauungssystem.

## Revendications

1. Procédé de préparation d'une composition d'oligosaccharides, comprenant:
la production d'une composition aqueuse qui comprend au moins un oligosaccharide et au moins un monosaccharide par saccharification de l'amidon;
le fractionnement de la composition aqueuse par une méthode comprenant une chromatographie SSMB (en lit mobile simulé séquentiel) pour former un flux riche en monosaccharides et un flux riche en oligosaccharides; et
la récupération du flux riche en oligosaccharides.

2. Procédé selon la revendication 1, dans lequel la composition aqueuse comprend du dextrose, du fructose, et un mélange d'oligosaccharides.

3. Procédé selon la revendication 1, dans lequel le flux riche en oligosaccharides comprend au moins environ 50% en poids d'oligosaccharides exprimé en matière sèche.

4. Procédé selon la revendication 3, dans lequel le flux riche en oligosaccharides comprend au moins environ 90% en poids d'oligosaccharides exprimé en matière sèche.

5. Procédé selon la revendication 1, dans lequel le fractionnement comprend une filtration sur membrane.

6. Procédé selon la revendication 1, dans lequel le fractionnement comprend une nanofiltration.

7. Procédé selon la revendication 1, dans lequel le flux riche en oligosaccharides comprend une quantité mineure de dextrose et de fructose, et dans lequel le procédé comprend en outre la mise en contact du flux riche en oligosaccharides avec une enzyme d'isomérisation de sorte qu'au moins une certaine quantité du dextrose est converti en fructose, produisant ainsi un flux riche en oligosaccharides isomérisé.

8. Procédé selon la revendication 1, comprenant en outre une filtration sur membrane du flux riche en oligosaccharides pour produire un second flux riche en monosaccharides et un second flux riche en oligosaccharides.

9. Procédé selon la revendication 8, dans lequel le second flux riche en oligosaccharides comprend plus de 90% environ en poids d'oligosaccharides exprimé en matière sèche.

10. Procédé selon la revendication 1, dans lequel le flux riche en oligosaccharides comprend une quantité mineure de monosaccharides, et dans lequel le procédé comprend en outre l'hydrogénation du flux riche en oligosaccharides pour convertir au moins une certaine quantité des monosaccharides qu'il contient en alcools, produisant ainsi un flux riche en oligosaccharides hydrogéné.

11. Procédé selon la revendication 8, dans lequel le second flux riche en oligosaccharides comprend une quantité mineure de monosaccharides, et dans lequel le procédé comprend en outre l'hydrogénation du second flux riche en oligosaccharides pour convertir au moins une certaine quantité des monosaccharides qu'il contient en alcools, produisant ainsi un flux riche en oligosaccharides hydrogéné.

12. Procédé selon la revendication 1, comprenant en outre la mise en contact du flux riche en oligosaccharides avec une enzyme glucosidase de sorte qu'au moins une certaine quantité des éventuels monosaccharides résiduels présents dans le flux se lient par covalence à des oligosaccharides ou d'autres monosaccharides.

13. Procédé selon la revendication 1, comprenant en outre la réduction de la couleur du flux riche en oligosaccharides par mise en contact de celui-ci avec du charbon actif.

14. Procédé selon la revendication 1, dans lequel le flux riche en oligosaccharides est capable d'être digéré lentement par l'appareil digestif humain.

15. Procédé selon la revendication 1, dans lequel le fractionnement comprend une nanofiltration, la composition aqueuse comprend du dextrose, du fructose, et un mélange d'oligosaccharides, et le flux riche en oligosaccharides comprend une quantité mineure de dextrose et de fructose, et dans lequel le procédé comprend en outre au moins une des étapes suivantes:
mise en contact du flux riche en oligosaccharides avec une enzyme d'isomérisation de sorte qu'au moins une certaine quantité du dextrose est convertie en fructose;
filtration sur membrane du flux riche en oligosaccharides;
hydrogénation du flux riche en oligosaccharides pour convertir au moins une certaine quantité des monosaccharides qu'il contient en alcools;
mise en contact du flux riche en oligosaccharides avec une enzyme glucosidase pour créer un produit d'inversion de sorte qu'au moins une certaine quantité des éventuels monosaccharides résiduels présents dans le flux se lie par covalence à des oligosaccharides ou d'autres monosaccharides; et
réduction de la couleur du flux riche en oligosaccharides par mise en contact de celui-ci avec du charbon actif;
dans lequel le flux riche en oligosaccharides est capable d'être digéré lentement par l'appareil digestif humain.
